(19) Europäisches Patentamt
European Patent Office
Office européen des brevets

(11) Veröffentlichungsnummer: **0 244 832 B1**

(12) **EUROPÄISCHE PATENTSCHRIFT**

(45) Veröffentlichungstag der Patentschrift: **24.06.92**

(51) Int. Cl.[5]: **A61K 31/20**, A61K 47/00

(21) Anmeldenummer: **87106520.7**

(22) Anmeldetag: **06.05.87**

# (54) Ungesättigte Fettsäuren enthaltende Zubereitungen für die Synthese von Prostaglandinen und Hydroxyfettsäuren in biologischen Systemen.

(30) Priorität: **09.05.86 DE 3615710**

(43) Veröffentlichungstag der Anmeldung:
**11.11.87 Patentblatt 87/46**

(45) Bekanntmachung des Hinweises auf die Patenterteilung:
**24.06.92 Patentblatt 92/26**

(84) Benannte Vertragsstaaten:
**AT BE CH DE ES FR GB GR IT LI LU NL SE**

(56) Entgegenhaltungen:
**EP-A- 0 090 378**
**WO-A-84/02271**
**DE-A- 3 226 232**
**FR-A- 2 231 379**

(73) Patentinhaber: **HOECHST AKTIENGESELLSCHAFT**
**Postfach 80 03 20**
**W-6230 Frankfurt am Main 80(DE)**

(72) Erfinder: **Weithmann, Klaus Ulrich, Dr.**
**Am Domherrnwald 18**
**W-6238 Hofheim am Taunus(DE)**

EP 0 244 832 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Langkettige, mehrfach ungesättigte Fettsäuren bzw. deren Ester und andere Derivate haben Bedeutung als Ausgangsmaterial für die enzymkatalysierte Herstellung von Prostaglandinen und Hydroxyfettsäuren. So können beispielsweise mit Hilfe von Zellen oder von Zellhomogenaten bzw. Zellfraktionen insbesondere aus den Substraten Dihomogammalinolensäure, Arachidonsäure bzw. Eicosapentaensäure die sonst nur in aufwendigen chemischen Verfahren herstellbaren Produkte auf biochemischem Wege präparativ gewonnen werden. Bei solchen präparativen Fragestellungen wird man eine Stimulierung der Bildung des gewünschten Prostaglandins anstreben. Dies kann durch Zusatz von geeigneten Enzym-Cofaktoren erreicht werden. Die genannten Fettsäuren lassen sich aber auch für medizinische Zwecke einsetzen, wenn man z.B. die Fähigkeit von Körperzellen, bzw. Zellhomogenaten oder Zellfraktionen (z.B. Keratinozyten; durch Biopsie gewonnene Zellen der Blutgefäße; Zellen oder Homogenate der Nierenmedulla), die betreffende Fettsäure zu metabolisieren bzw. zu Prostaglandinen mit bestimmter biologischer Wirkung umzusetzen, klinisch überprüft. Bei solchen medizinischen Fragestellungen kommt es nicht auf die Einheitlichkeit des gebildeten Prostaglandins an, aber man wird bestrebt sein, die Bildung des als medizinische Meßgröße dienenden Prostaglandins zu stimulieren.

Die vorliegende Erfindung beschreibt neue, haltbare Zubereitungen, die langkettige, mehrfach ungesättigte Fettsäuren enthalten, mit deren Hilfe sich vorteilhaft Prostaglandine und Hydroxyfettsäuren herstellen lassen, die für die genannten Zwecke verwendet werden können.

Es ist bereits bekannt, daß die Umsetzung von mehrfach ungesättigten Fettsäuren, wie Arachidonsäure, durch prostaglandinsynthetisierende Enzyme mit Hilfe von gewissen Cofaktoren stimuliert und dabei auch die Synthese ganz bestimmter Prostaglandine angeregt werden kann. Z.B. ist in Biochemical Pharmacology 31 (1982) 3591 ein in vitro System beschrieben, das Arachidonsäure mit Hilfe des aus Schafsamenblasen gewonnenen Enzyms jeweils mit oder ohne Zusatz der Cofaktoren Glutathion und Hydrochinon umsetzt. In loc cit, Seite 3595 ist erläutert, daß die Umsetzung durch die genannten Cofaktoren beschleunigt wird. Ein ähnliches System aus Arachidonsäure, Homogenat aus der Nierenmedulla des Kaninchens, Glutathion und Hydrochinon, wird in Life Sciences 26 (1980) 765 bechrieben.

In der WO-A-84/02271 wird ein Gemisch beschrieben, das polyungesättigte Fettsäuren und Stabilisatoren enthält. Als Stabilisatoren werden u.a. Vitamin E und Glycerin genannt.

Die DE-A-32 26 232 beschreibt ebenso ein Gemisch aus ungesättigten Fettsäuren, das zusätzlich auch $\alpha$, $\beta$ oder $\gamma$ -Cyclodextrin, d.h. sogenannte Stabilisatoren enthält.

Schließlich offenbart auch die FR-A-2 231 379 ein Gemisch aus polyungesättigten Fettsäuren und Vitamin E, einen Stabilisator.

Es wurden nun für die vorher genannten Zwecke wäßrige Reagenzzubereitungen hergestellt, die die mehrfach ungesättigten Fettsäuren vorzugsweise kombiniert mit stimulierenden Enzym-Cofaktoren enthalten. Derartige Zubereitungen sind nicht nur neu, sondern sie zeigen auch unerwartete Eigenschaften. Wie weiter unten im einzelnen aufgeführt, verändern diese Stimulatoren ausnahmslos die Haltbarkeit der Fettsäuren in überraschender Weise. So können sich innerhalb kurzer Zeit deutlich mehr unerwünschte Zersetzungs- und Umwandlungsprodukte der Arachidonsäure in Glutathion bzw. Hydrochinon enthaltenden Arachidonsäure-Zubereitungen bilden, als dies in Zubereitungen der Fall ist, die diese Zusätze nicht enthalten. Diese Nebenprodukte sind hochreaktiv und können unerwünschte, auch biologische, Nebenreaktionen auslösen. Die unten näher beschriebenen eigenen Untersuchungen zeigen aber, daß die Prostaglandinsynthese auch durch Gemische, die mehrfach ungesättigte Fettsäuren, wie Arachidonsäure, zusammen mit anderen schwefelhaltigen Verbindungen, wie dem Disulfid Liponsäure, oder den reduzierten Thiolen Homocystein und Cystein, bzw. deren am Stickstoffatom acylierten Derivate, enthalten, stimuliert werden kann, wobei sich erfindungsgemäß viel weniger unerwünschte Nebenprodukte bilden. Dies ist überraschend, da mit anderen in der Natur vorkommenden oder von natürlichen Verbindungen abgeleiteten Schwefelverbindungen, wie Coenzym A, bzw. dessen Spalt- oder Hydrolyseprodukte Cysteamin und Pantethein, bzw. Dihydroliponsäure, Cystin und Mono- oder Polythiolzuckerverbindungen, wie Dithiothreitol bzw. Dithioerythrit diese vorteilhaften Effekte nicht erzielt werden konnten. Im experimentellen Teil ist ausgeführt, daß die unerwünschten Nebenreaktionen durch Zusatz von Liponsäure völlig, durch Zusatz der anderen erfindungsgemäßen schwefelhaltigen Verbindungen weitgehend, aber nicht völlig unterdrückt werden können.

Es ist nun schon bekannt, daß die Arachidonsäureumsetzung, bzw. die Synthese bestimmter Prostaglandine, auch durch Zusatz von Substanzen ohne Schwefelgehalt stimuliert werden kann, z.B. wie oben zitiert mit Hilfe von Hydrochinon, aber auch mit Phenol, Adrenalin, Noradrenalin, Tryptophan, 5-Hydroxytryptophan und Ascorbinsäure. Überraschenderweise wurde nun gefunden, daß der unerwünschte Zerfall von mehrfach ungesättigten Fettsäuren z.B. von Arachidonsäure, in wäßrigem Medium durch diese

Stimulatoren erheblich beschleunigt wird. Die Verwendung von z.B. 2-Butanol oder Pyridin statt Wasser, wirkt zusätzlich destabilisierend, während bei Verwendung von Dimethylsulfoxid, bzw. Äthanol, bzw. Polyolen wie Glycerin oder Polyäthylenglykol, die unerwünschten Nebenprodukte überraschenderweise nicht auftreten. Organische Lösungsmittel haben aber den Nachteil, daß sie Enzyme denaturieren; sie müssen also vor der Enzymreaktion entfernt oder mit Wasser verdünnt werden. Die nach dem Entfernen des Lösungsmittels verbleibenden Rückstände, die aus einem innigen Gemisch aus ungesättigter Fettsäure und Stimulator bestehen, sind überraschenderweise wieder instabil. Es ist noch nicht beschrieben worden, daß überraschend vorteilhafte wäßrige Zubereitungen für die Stimulierung der Prostaglandinsynthese mit Tyrosin erhalten werden können. Wie im experimentellen Teil ausgeführt, sind Zubereitungen, die ungesättigte Fettsäuren und Tyrosin oder Tyrosin-ähnliche Verbindungen enthalten, überraschenderweise völlig stabil.

Aber auch wäßrige Gemische bzw. Lösungen aus ungesättigter Fettsäure und den anderen oben genannten Stimulatoren können vollständig stabilisiert werden und zwar durch Zusatz von wasserlöslichen bzw. in Wasser emulgierbaren Lipiden oder von z.B. aus Human- oder Säugetierblut herstellbaren Proteinen, wie Serumalbumin oder Globuline, wie Gammaglobulin, oder porphyrinhaltigen Proteinen wie Hämoglobin, Methämoglobin oder Cytochrome. Erfindungsgemäß geeignet sind auch Vitamine der E-Reihe sowie Flavonoide, insbesondere solche mit Zuckeranteil, z.B. Rutin, oder Troxerutin (zur Nomenklatur siehe "The Merck Index", 10. Aufl. 1983, Rahway, N.J., USA), wohingegen Zucker ohne Flavonanteil, wie Rhamnoglucose, Glucose und Sorbit, nicht stabilisieren. Mit Hilfe von Cyclodextrinen lassen sich die erfindungsgemäßen Zubereitungen aber überraschenderweise herstellen.

Hinsichtlich der Stabilisierung mit Serumalbuminen ist zwar an sich bekannt, daß Albumine langkettige Fettsäuren auf energetisch niedrigem Niveau binden können, umso überraschender ist es aber, daß sich die derart gebundenen Fettsäuren in den erfindungsgemäßen Reagenzzubereitungen wie frei gelöste Fettsäuren verhalten, d.h. enzymatisch ungehindert umgesetzt werden. Es ist auch schon beschrieben worden (vgl. z.B. Journal of Biological Chemistry 253 (1978) 5061), daß die Enzyme der Prostaglandinsynthese Häm oder andere Porphyrine als prosthetische Gruppen gebunden haben. Dazu bedarf es aber nur kleinster Mengen an porphyrin haltigen Enzymen, die keine stabilisierende Wirkung auf die zugesetzten Mengen an ungesättigter Fettsäure haben. Ein Hinweis, daß mit einem größeren Anteil an porphyrinhaltigen Proteinen ein stabilisiertes Reagenz zur Stimulierung der enzymkatalysierten Prostaglandinsynthese hergestellt werden kann, findet sich in der Literatur nicht.

Stabile Zusammensetzungen, die aus Fettsäuren mit 20 bis 22 C-Atomen mit jeweils 3 oder mehr Doppelbindungen und als Stabilisatoren bis zu 30 Gewichtsprozent-Anteil Phospholipide, z.B. Lezithin, enthalten, sind in DE 34 197996 A1 offenbart. Solche stabilen Zubereitungen können ebenfalls in Kombination mit den bereits genannten Stimulatoren erfindungsgemäß verwendet werden.

Wie im experimentellen Teil ausgeführt, ist es jedoch zweckmäßiger, wenn die Zubereitungen aus den ungesättigten Fettsäuren mit 18 bis 22 C-Atomen mit mehr als 30 Gewichtsprozent Phospholipid, z.B. Lezithin, versetzt werden, weil dann stabilere Mischungen erhalten werden. Vorteilhaft ist eine bis zu 90 Gewichtsprozent Phospholipid, vorzugsweise 50 bis 80 Gewichtsprozent Phospholipid enthaltende Zubereitung. Im Hinblick auf medizinische Wirkungen ist eine Zubereitung, die die erfindungsgemäßen Fettsäuren mit 18 C-Atomen enthält, bevorzugt. Eine ungesättigte Fettsäuren enthaltende Zubereitung mit einem höheren als 30 gewichtsprozentigem Anteil an Phospholipiden ist bereits als Diätetikum vorgeschlagen worden (EP 0 148303 A1). Stabilisierende Effekte der Phospholipide auf die ungesättigten Fettsäuren werden in EP 0 0148303 A nicht offenbart. Es findet sich dort auch kein Hinweis darauf, daß die in der vorliegenden Anmeldung beanspruchten Zubereitungen bestehend aus ungesättigten Fettsäuren, Phospholipiden und Stimulatoren sich vorteilhaft für die in der vorliegenden Anmeldung beanspruchten Verwendungen, insbesondere für die beanspruchten medizinischen Anwendungen, eignen.

Gegenstand der Erfindung sind daher feste oder flüssige Stoffgemische oder Lösungen, enthaltend

A) eine oder mehrere ungesättigte Fettsäure(n), gekennzeichnet durch drei bis fünf isoliert angeordnete Doppelbindungen und 18 bis 22 geradkettig angeordnete Kohlenstoffatome, die an einem oder an zwei C-Atomen der Position 2, 3, 4, 16, 17, 18, 19, 20 methyliert oder äthyliert sein können, als freie Carbonsäure mit endständigem $-CO_2H$ oder als Carbonsäureamid oder $-CO_2X$, wobei X eine unter sauren Bedingungen abspaltbare Schutzgruppe, wie ein Alkylrest, z. B. ein Äthyl- oder Propylrest, oder ein 1- oder 2-Lysophospholipid, z.B. 2-Lysolecithin oder ein Metall- oder Amin-Kation oder die kationische Form eines Ionenaustauschers darstellt,

B) als Stimulatoren mit z.T. gleichzeitig stabilisierender Wirkung eine oder mehrere Verbindungen der folgenden Gruppen:

$B_1$) eine phenolische Verbindung der Formel I

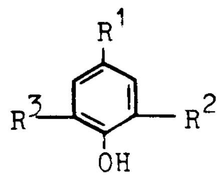

I

in der die Reste $R^2$ und $R^3$ Hydroxylgruppen, oder Wasserstoff und $R^1$ den Rest -OH, $-CO_2H$, $-CH_2-CO_2H$,$-CH=CH-CO_2H$, $-CH_2-CHR^4R^5$, $-CHOH-CH_2-NH-R^6$ mit $R^4$ = -H oder $-CO_2H$ und $R^5$ = -H oder $-NH_2$ und $R^6$ = -H, $-CH_3$ oder $-C_2H_5$ bedeuten,

$B_2$) ein Indolderivat der Formel II

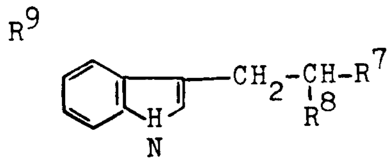

II

in der $R^7$ Wasserstoff oder -COOH, $R^8$ Wasserstoff oder $-NH_2$ und $R^9$ Wasserstoff oder -OH bedeuten,

$B_3$) Cystein oder Homocystein, oder Liponsäure, deren acyclischer Alkylrest um bis zu vier Methylengruppen verkürzt sein kann,

$B_4$) ein Peptid, bestehend aus maximal zehn Aminosäuren, bei dem eine oder mehrere der Aminosäuren durch jeweils eine der Verbindungen gemäß $B_1$) bis $B_3$) ersetzt ist,

$B_5$) eine Verbindung gemäß $B_1$) bis $B_4$), die an einem N-Atom eine $C_1$-$C_4$-Alkanoylgruppe tragen kann,

$B_6$) einen Flavonabkömmling, der mit wenigstens einer Hydroxylgruppe, die einen Zuckerrest trägt substituiert ist,

$B_7$) ein Salz der ionischen Formen der Verbindungen gemäß $B_1$)-$B_6$) und gegebenenfalls

$B_8$) eine Carbonsäure verbindung gemäß $B_1$) bis $B_7$), die mit einem Alkoxy-Rest verestert sein kann oder als Carbonsäureamid vorliegt, das auch mono- oder dialkyliert sein kann; und gegebenenfalls

c) als Stabilisatoren eine oder mehrere Verbindungen der folgenden Gruppen:

$C_1$) Dimethylsulfoxid, Äthylalkohol, Glycerin polyester glycerin, Äthylenglykol, Polyäthylenglykol oder Glycerintriacetat,

$C_2$) Phospholipide, Zuckerlipide, Cyclodextrine, Proteine, z.B. solche aus Human- und Säugetierblut herstellbare, Vitamine der E-Reihe oder Flavonabkömmlingen ohne Zucherrest

als haltbare Zubereitung für die Synthese von Prostaglandinen und Hydroxyfettsäuren in biologischen Systemen.

Als Metallkationen X können z.B. solche der Alkalime wie Lithium, Natrium und Kalium, und der Erdalkalime wie Magnesium und Calcium, aber auch kationische Form derer Metalle, wie Aluminium, Zink und Eisen verwende werden, gegebenenfalls chelatisiert mit Zitronensäure Äthylendiamintetraessigsäure und dgl. Als Aminkatione nen solche von primären, sekundären oder tertiären Aminen wie der Alkylamine, z.B. Mono-, Di-, und Trimethyl, bzw. -äthyl-, -propyl-, -isopropyl-, -butyl-, -isobutyl-, -t-butyl-, sowie N-(Methylhexyl)-, N-Methyl-hexyl-, Benzyl-, $\beta$-Phenyl-äthylamin, Äthylendiamin, Diäthylentriamin, Pyrrolidin, Piperidin, Morpholin, Piperazin, Mono-, Di- und Triäthanolamin, Äthyldiäthanolamin, N-Butyläthanolamin, Tris-(hydroxymethyl)-aminomethan, und dergleichen. Geeignete Aminsalze sind z.B. die des Tryptamins, Cysteins sowie die basischen Aminsalze des Lysins und des Arginins. Geeignete quaternäre Ammoniumkationen sind z.B. das Tetramethylammonium, Tetraäthylammonium und das Benzyltrimethylammonium. Diese Kationen können auch zur Salzbildung der anionischen Formen der Verbindungen gemäß $B_1$) bis $B_6$) verwendet werden, wohingegen zurSalzbildung bei den kationischen Formen Chlorid und Fluorid bevorzugt sind.

Bevorzugt enzymatisch umgesetzt werden die Fettsäuren mit den Bezeichnungen 18:2ω-6, 20:4ω-6, 22:5ω-6, 18:3ω-3, 20:5ω-3, 22:6ω-3, 18:3ω-6, 20:3ω-6, 22:4ω-6 und 22:4 ω-3, wobei in der üblichen Nomenklatur die erste Zahl die Anzahl der Kohlenstoffatome, die Zahl nach dem Doppelpunkt die Anzahl der Doppelbindungen und die Zahl nach dem ω die Position der ersten Doppelbindung gerechnet vom Methylende des Moleküls bedeutet. Die Produkte bestimmter Fettsäuren sind bereits bekannt, und es ist ebenfalls bekannt, daß man durch Verwendung gewisser Stimulatoren die Geschwindigkeit der Umsetzung

der ungesättigten Fettsäure, sowie die Bildung bestimmter Produkte, anregen kann. Wie im experimentellen Teil näher ausgeführt wird, ermöglicht es die vorliegende Erfindung, die Bildung bestimmter Fettsäure-Produkte in vorteilhafter Weise zu stimulieren. Es wird also ganz von der jeweiligen Aufgabenstellung abhängen, wie man die Zusammensetzung der erfindungsgemäßen Zubereitung hinsichtlich Art der ungesättigten Fettsäure, Art des Stimulators, aber auch des Stabilisators, zu wählen hat. Soweit es sich bei den Stimulatoren um Aminosäuren bzw. Peptide handelt, können sowohl die L- als auch die D-Formen aber auch Gemische aus D- und L-Formen angewendet werden.

Stimulatoren der Formel I mit $R^2$ = $R^3$ = -H und $R^1$ = $-CH_2-CH(NH_2)-CO_2H$, sowie Peptide, die diese enthalten bzw. Liponsäure, deren Seitenkette verkürzt sein kann, bzw. Flavonoide, haben den besonderen Vorzug, daß sie die Haltbarkeit der Fettsäuren auch ohne zusätzlichen Stabilisator erhöhen.

Auch die anzuwendenden Mengen der ungesättigten Fettsäuren in den erfindungsgemäßen Zubereitungen sind von der gewünschten Verwendung abhängig. Für die präparative Herstellung von Prostaglandinen kann jede gewünschte Menge der Zubereitung portioniert werden. Für die Herstellung von erfindungsgemäßen Reagenzzubereitungen, die zur Verwendung bei klinisch-chemischen Aufgaben dienen, also z.B. zur Überprüfung der Fähigkeit von Zellen oder Zellbestandteilen, wie Blutzellen oder Biopsiematerial der Niere, der Lunge, des Magens, usw., ungesättigte Fettsäuren umzusetzen, bzw. Prostaglandine mit bestimmter biologischer Wirkung zu synthetisieren, genügen erfindungsgemäße Reagenzgemische, die 5 mg bis 1 pg, vorzugsweise 100 $\mu$g bis 1 ng ungesättigte Fettsäure enthalten. Die Reagenzien können als Suspension bzw. gelöst z.B. in Wasser, Äthanol, Äthylenglykol, Polyäthylenglykolen oder Glycerin oder Mischungen aus diesen Lösungsmitteln vorliegen. Nach Entfernen des Lösungsmittels liegen die Reagenzien in Form von Gemischen bzw. Vermengungen vor Die Entfernung der organischen Lösungsmitel ist insbesondere bei Verwendung der Reagenziengemische für enzymkatalysierte Umsetzungen in vitro erforderlich.

Aus biologischem Material gewinnbare Enzymsysteme zur Herstellung von Prostaglandinen sind an sich bekannt. Es ist auch bekannt, daß je nach Herkunft und Art des biologischen Materials verschiedene Prostaglandine synthetisiert werden können. Die erfindungsgemäßen Reagenzien können nun zur Herstellung dieser Prostaglandine verwendet werden. Dazu wird eine Lösung bzw. Suspension bzw. Homogenat des biologischen Materials mit den erfindungsgemäßen Zubereitungen gemischt und in bekannter Weise inkubiert. Durch die Wahl des Stimulators kann die Synthese bestimmter Prostaglandine bis zu einem gewissen Ausmaß gesteuert werden. Unerwünschte Fettsäure-Produkte, wie Thromboxan oder Leukotriene, können gemäß dem Stand der Technik durch Zusatz von Thromboxanhemmern bzw. Lipoxygenase-Hemmern inhibiert werden. Falls die ungesättigte Fettsäure im Reagenz in gebundener Form, z.B. als Ester, vorliegt, dann ist Sorge dafür zu tragen, daß sie vor bzw. während der enzymatischen Umsetzung in die freie Form überführt wird, gegebenenfalls durch die in biologischem Material ohnehin vorhandenen Lipase-Enzyme, bzw. durch Zusatz geeigneter Lipase-Enzyme, bzw. schon vor der enzymatischen Umsetzung durch die bekannten Verfahren der chemischen Hydrolyse. Nach Extraktion des gebildeten Prostaglandins, bzw. der gebildeten Prostaglandine mit Hilfe von geeigneten Extraktionsmitteln, z.B. Acetylacetat, kann das gewünschte Prostaglandin in reiner Form dargestellt werden, z.B. durch bekannte chromatographische Verfahren, wie Hochdruckflüssigkeitschromatographie. Die Mengenverhältnisse der erfindungsgemäßen Bestandteile in den Zubereitungen können in weiten Bereichen schwanken. Sie sind abhängig von der Art der ungesättigten Fettsäure, dem verwendeten Stimulator, bzw. Stabilisator und auch von der angestrebten Verwendung. Das Gewichtsverhältnis zwischen ungesättigter Fettsäure (auf die freie Säure bezogen) und Stimulator beträgt in allgemeinen zwischen 50 und 0,1, vorzugsweise zwischen 20 und 0,5. Die Menge des zugesetzten Stabilisators ist so besessen, daß sein Gewichtsanteil, auf die freie ungesättigte Fettsäure bezogen etwa zwischen 0,5 und 100, vorzugsweise zwischen 1 und 15 und insbesondere zwischen 2 und etwa 8 bis 10 liegt.

Biologische Systeme können auch in vivo mit den medizinisch geeigneten erfindungsgemäßen Zubereitungen behandelt werden, und zwar vorteilhafter als dies mit den einzelnen Komponenten der erfindungsgemäßen Zubereitungen möglich ist. Eine systemische (z.B. intravenöse) Verabreichung der erfindungsgemäßen Zubereitungen ist allerdings nicht sinnvoll, weil die ungesättigten Fettsäuren metabolisiert werden können, bevor sie den Wirkort erreichen. Direkt zugänglich sind dagegen z.B. die Zellen der Haut, des Mundes, der Speiseröhre, der Nase, der Augen, des Darmes und des Magens und des Bronchial- und Lungentraktes.

Prostaglandinmangelzustände von Menschen oder Tieren, z.B. in Magen-Darm-Bereich, die z.B. zu gestörter Magensaftsekretion und zu gastrointestinalen Läsionen (Geschwüren) führen, können z.B. durch erfindungsgemäße Zubereitungen in Form von oralen (peroralen) Arzneiformen behandelt werden. Der Darm kann auch mit enterisch (magensäureunlöslich) überzogenen Arzneiformen behandelt werden. Falls die Herbeiführung von Durchfall, z.B. bei Verstopfungen erwünscht ist, können auch Arzneimittelformen für die rektale Therapie angewendet werden, während für die Auslösung der Wehen, z.B. zur Einleitung des

Geburtsvorganges, Arzneiformen für die intravaginale Therapie angewendet werden können. Fehlfunktionen der Haut und des darunter liegenden Bindegewebsbereiches können durch erfindungsgemäße dermale Zubereitungen behandelt werden. Orale Zubereitungen und Suppositorien für die Therapie am Menschen sind so bemessen, daß sie pro Dosierungseinheiten zwischen 0,5 u. 2000 mg, im allgemeinen zwischen 2 und 1000 mg und vorzugsweise zwischen 5 u. 250 mg an ungesättigter Fettsäure enthalten. Bei besonders therapieresistenten Krankheiten, z.B. bei hartnäckiger Verstopfung kann es auch angezeigt sein, eine Dosierungseinheit bis zu 6000 mg oder mehr an ungesättigter Fettsäure anzuwenden. Überraschenderweise sind die erfindungsgemäßen geradkettigen Fettsäuren mit 18, ferner auch die mit 22 C-Atomen ebenso wirksame Therapeutika für Magen-Darm-Geschwüre wie die 20 C-Atome enthaltenden. Dies ist von medizinischer Bedeutung, weil sie weniger Nebenwirkungen, wie Durchfall, auslösen. Eine geeignete Therapie besteht z.B. in der Verabreichung von einer, zwei oder mehr, vorzugsweise 3 bis 8 Einzeldosierungen pro Tag mit der erfindungsgemäßen Zubereitung, wobei die erforderliche Menge von der Zahl der Einzeldosierungen und auch der zu behandelnden Krankheit abhängig ist und eine Dosierung z.B. auch aus mehreren, gleichzeitig verabreichten Einzeldosierungen bestehen kann.

Es ist bekannt, daß als unerwünschte Nebenwirkungen von gewissen Arzneimitteln gastrointestinale Unverträglichkeiten, die zu gastrointestinalen Geschwüren führen, auftreten können. Zur Verhinderung bzw. zur Therapie solcher gastrointestinalen Schleimhautschäden sind die erfindungsgemäßen Zubereitungen ebenfalls geeignet. Die Behandlung mit den Zubereitungen kann sowohl vor als auch gleichzeitig oder nach der Gabe der schleimhautschädigenden Medikamente erfolgen. Besonders vorteilhaft ist es, die erfindungsgemäßen Reagenzien mit den magenschleimhautschädigenden Medikamenten in einer geeigneten pharmazeutischen Zubereitung zu kombinieren. Dabei können die Komponenten sowohl kompartimentiert, aber auch als Gemisch vorliegen. Zahlreiche Arzneimittel, die zu Magenunverträglichkeiten bzw. gastrointestinalen Läsionen führen, sind bekannt. Dazu gehören Antirheumatika (steroide und nichtsteroide Antiphlogistika), wie Corticosteroide (z.B. Cortison), Salicylsäurederivate (z.B. Acetylsalicylsäure, Diflunisal) oder Phenylalkansäuren (z.B. Piroxicam, Acemetacin, Pimetacin, Nambumeton, Carprofen, Pirprofen, Fenclofenac, Sulindac, Indometacin, Fenoprofen, Tiaprofensäure, Tolmetin, Flurbiprofen, Suprofen, Indoprofen, Ibuprofen, Naproxen, Alclofenac, Ketoprofen, Diclofenac) oder Pyrazolone (Phenylbutazon, Metamizol), aber auch Blutdrucksenker (z. B. Reserpin), Vasodilatatoren auf Xanthinbasis (z. B. Pentoxifyllin), tuberkulosehemmende Mittel (z. B. Rifampicin) oder Zytostatika (z. B. Methohexat).

Die als pharmazeutische Präparate medizinisch verwendeten erfindungsgemäßen Zubereitungen können die üblichen pharmazeutischen Hilfs- und Trägerstoffe enthalten, die zur Zubereitung von oral und dermal (auch z.B. nasal, sublingual, rektal und intravaginal) wirksamen Arzneimitteln geeignet sind. Dazu gehören beispielsweise Stärke, Saccharose, Calciumsulfat, Gelatine, Stärkepaste, Sirup, Weizen- und Maisstärke, Stearinsäure, Stearatsalze, Zucker, Saccharin, Nahrungsmittelbestandteile für Tiere wie Fette, Kohlenhydrate, Proteine und mineralische Feststoffe; Konservierungsmittel, Süßstoffe, Farbstoffe, Gewürzstoffe oder Suspensionsmittel, mineralische und pflanzliche Öle wie Erdnuß-, Oliven-, Nachtkerzen-, Soja-, Sonnenblumen-, Baumwollsamen-, Fisch- und Sesamöl bzw. Fraktionen dieser Öle, sowohl ionische als auch nichtionische Detergenzien (z.B. wie unten beschrieben), Konservierungsstoffe und Antifungusmittel wie Parabene, Chlorbutanol, Benzylalkohol, Phenol, Thimerosal, isotonische Mittel wie Zucker oder Natriumchlorid. Die mehrfach ungesättigten Fettsäuren können auch an Polydextran oder Polyacrylamid-, Alkylsulfat- oder -phosphat-Gele, oder an Aluminiumhydroxid oder Kieselsäure adsorbiert, bzw. damit vermischt sein.

Die Präparate für die dermale Verabreichung sind beispielsweise wäßrige, alkoholische oder Polyole enthaltende Lösungen oder Suspensionen, ölige Lösungen oder Suspensionen, Pulver für die anschließende Einarbeitung in eine dermal anwendbare Form durch Zugabe des erforderlichen Trägers. Die Lösungen oder Suspensionen werden mit dem erforderlichen pharmazeutischen, z.B. Fette, Wachse, Vaseline oder Paraffin enthaltenden Hilfs- und Trägerstoffen und Konservierungsmitteln, Suspensions- und Dispersionsmitteln und isotonischen Mitteln, wie z.B. Methyl- und Propylparabenen, Natriumchlorid, Polyäthylenglykolen, insbesondere Polyäthylenglykol 4000, Natriumcarboxymethylcellulose, Natriumalginat, Polyvinylpyrrolidon, Sorbimacrogololeat, Kondensationsprodukt von Äthylenoxid mit Fettsäuren, z.B. Polyoxyäthylenstearat oder mit Fettalkoholen wie z.B. Heptadecaäthylenoxycetanol, oder mit partiellen Estern wie z.B. Polyoxyäthylensorbit-monooleat, oder Hexitanen, die von Sorbit stammen wie z.B. Polyoxyäthylen-sorbitan-monooleat formuliert. Suspensionen in öligen Medien werden hergestellt, indem man den Wirkstoff in den o.g. Ölen dispergiert. Diese Suspensionen können Mittel enthalten, die die Absorption verzögern wie z.B. Aluminiummonostearat. Ein trockenes, z.B. gefriergetrocknetes Präparat, das zum gewünschten Zeitpunkt mit einem geeigneten pharmazeutischen Träger vermischt werden kann, stellt eine weitere Ausführungsform dar. Präparate des Wirkstoffs, wie z.B. Tabletten, Kapseln, Suppositorien, Sirupe, Lösungen, Suspensionen und Elixiere enthalten pharmazeutische Hilfsstoffe, die die Präparate für diese Verabreichungsformen zur

Erzielung der medizinischen Wirkung geeignet machen. Die erfindungsgemäßen Mischungen werden mit pharmazeutischen Hilfsstoffen wie Lactose, Stärke, Akaziengummi, Gelatine, Talkum und dergleichen vermischt und zu Kapseln, Tabletten und dgl. oder zu üblichen Suppositorienmassen verarbeitet, z.B. solche auf Triglyceridbasis, wie z.B. Witepsol-Suppositorienmassen (H.P. Fiedler, Lexikon der Hilfsstoffe für Pharmazie, Kosmetik und angrenzende Gebiet, 1971, Bd. 9., S. 548-50 und 632-634 oder für intravaginale Suppositorien US 263026 und 663145); gegebenenfalls Fettalkohole; feste Kohlenwasserstoffe, wie Vaseline oder Paraffin solidum; gesättigte Fettsäuren, wie Laurinsäure, Myristinsäure, Palmitinsäure, Stearinsäure; Emulgatoren, wie ethoxylierte Triglyceride, polyethoxylierte pflanzliche Öle; Fettsäurezuckerester; Silikone; Gelatine; Methylcellulose; Hydroxypropoxycellulose, Hydroxypropylcellulose; Polyethylenglykole; Polyvinylpyrrolidon; Polyvinylalkohol; Polyacrylsäure und deren Salze gemischt, enthaltend, oder mit Wasser, Suspensionsmitteln, Saccharose, Konservierungsstoffen oder hydro-alkoholischen Trägern vermischt und zu Suspensionen, Lösungen oder Elixieren verarbeitet. Es kann erwünscht sein, daß die ungesättigte Fettsäure langsam oder verzögert aus der pharmazeutischen Zubereitung freigesetzt wird. Eine solche verzögerte Freisetzung kann entsprechend dem Stand der Technik erreicht werden. Es hängt vom pH des biologischen Mediums ab, ob man die verzögerte Freisetzung mit z.B. Polystyrol oder Polyacrylderivaten (Eudragit$^{(R)}$) oder z.B. durch Bindung an einen geeigneten Ionenaustauscher erreicht. Es können auch enterisch überzogene Präparate beispielseise als Tabletten, Kapseln, Pillen, Kügelchen oder Mikrokügelchen hergestellt werden. Sie liegen in Form von Dosierungseinheiten vor, z.B. als einzelne Tablette oder Kapsel oder in Form einer Kollektion von Pillen, Mikrokügelchen oder Mikrosphären, die enterische Eigenschaften und verlängerte Wirkung besitzen. Solche enterisch überzogenen Präparate und die dabei verwendeten Überzugsstoffe sind in der Literatur beschrieben, z.B. in den US-PS 20 93 462 und 21 96 768 (Zelluloseacetatphthalat), 28 97 121 (Copolymeres aus Styrol und Maleinsäure), 30 81 233 und weitere.

Experimenteller Teil:

Methode 1: Überprüfung der Haltbarkeit der ungesättigten Fettsäuren in den Zubereitungen

Die stabilisierende Wirkung von Substanzen auf mehrfach ungesättigte Fettsäuren wurde wie folgt überprüft:

Test A für wasserlösliche Substanzen:

0,125 mg ungesättigte Fettsäure, bzw. Na-Salz (s. Tabellen) (Natriumarachidonat, Fa. Sigma München, Deutschland) werden in 0,05 ml Wasser gelöst. Hierzu werden 0,2 ml wässrige Lösung, die 2 mg Stabilisator enthält und zusätzlich 2 mg Stimulator enthalten kann, gemischt (vgl. Tab. 1), hierauf sofort 0,1 ml entnommen, extrahiert und analysiert (s. unten). Weitere Bearbeitung nach Modifikation 1 oder 2.

Modifikation 1 für wässrige Lösungen

Nach 24-stündiger Inkubation bei 37 °C werden wieder 0,1 ml entnommen, extrahiert und analysiert (s. unten).

Modifikation 2 für feste Gemische (Lyophilisate)

Die Testlösung wird lyophilisiert, das Lyophilisat 24 Stunden bei 37 °C inkubiert, dann wieder in der vorherigen Wassermenge gelöst, 0,1 ml entnommen, extrahiert und analysiert (s. unten).

Test B für nicht wasserlösliche Substanzen:

0,125 mg ungesättigte Fettsäure (Arachidonsäure; 5,8,11,14,17-Eicosapentaensäure; 8,11,14-Eicosatriensäure, Fa. Sigma, München) werden in 0,05 ml Äthanol oder Dimethylsulfoxid gelöst. Hierzu werden 0,2 ml Äthanol (bzw. Dimethylsulfoxid), das 2 mg Stabilisator enthält und zusätzlich 2 mg des Stimulators enthalten kann (vgl. Tab. 1), gegeben, sofort 0,1 ml entnommen und wie unten extrahiert und analysiert. Weitere Behandlung nach Modifikation 1 oder 2.

Modifikation 1 für Lösungen

Nach 24-stündiger Inkubation bei 37 °C werden wieder 0,1 ml entnommen, mit 0,1 ml Wasser gemischt,

extrahiert und analysiert.

Modifikation 2 für feste Gemische

Die restliche Lösung wird vom Lösungsmittel befreit, 24 Stunden bei 37 °C inkubiert, dann wieder in 0,15 ml Lösungsmittel gelöst, 0,1 ml entnommen, mit 0,1 ml Wasser gemischt, extrahiert und analysiert.

Extraktion und Analyse

Die erhaltenen Proben werden mit 0,05 ml 1,2 M wässriger Zitronensäurelösung versetzt, zweimal mit je 0,5 ml Acetylacetat extrahiert, das Acetylacetat aus den vereinigten Extrakten entfernt, und der Rückstand in 0,8 ml Methanol gelöst und mittels Hochdruckflüssigkeitschromatographie (HPLC) analysiert (Säule Rad Pak C-18 100 x 8 mm der Fa. Waters, Königstein, Deutschland, 1,5 ml/min., Laufmittel 800 ml Methanol, 200 ml Wasser, 0,1 ml Eisessig). Die Trennung wird mit einen handelsüblichen UV-Detektor bei 206 mm verfolgt.

Methode 2: Prostaglandinsynthese

0,3 ml 0,03 M Kaliumphosphatpuffer pH = 8,0 enthalten 0,5 mg Enzym (als Schafsamenblasenvesikeln )(Biochemistry 10 (1971) 2372) oder Homogenat der inneren Nierenmedulla (Life Sciences 26 (1980) 765), 2,75 µg 14-C-Arachidonsäure (0,5 µCi) und 0,5 mg Stimulator und gegebenenfalls 0,5 mg Stabilisatorsubstanz (s. Tab. 1). Nach 10 Minuten Inkubation bei 37 °C wird mit 0,05 ml 1,2 M Zitronensäure abgestoppt, zweimal mit je 0,5 ml Acetylacetat extrahiert, die vereinigten Extrakte vom Lösungsmittel befreit, der Rückstand in 0,2 ml Methanol aufgelöst, 1:1 mit Wasser verdünnt und über HPLC aufgetrennt (Säule Nucleosil C-18, 125 x 4,6 mm, Fa. Bischoff, Leonberg, Deutschland, Laufmittel 300 ml Acetonitril, 700 ml Wasser, 1 ml Eisessig, nach 30 min. reines Methanol). Die Auftrennung der radioaktiven Fraktionen wird mit einem Monitor (LB 504 der Fa. Berthold, Wildbad, Deutschland) verfolgt. Die gebildeten Produkte, wie 6-Keto-Prostaglandin-$F_{2alpha}$, Prostaglandin $E_2$, Prostaglandin-$F_{2alpha}$, Prostaglandin $D_2$, werden unter diesen Bedingungen aufgetrennt.

Tabelle 1 Stimulierung der Prostaglandinbildung in stabilisierten Fettsäurezubereitungen

Die Prostaglandine $PGE_2$, $PGF_{2alpha}$ und $PGD_2$ als Produkte der Arachidonsäure wurden mit Hilfe der Methode 2 quantifiziert, und die Summe als "Gesamtprostaglandin" auf 100% bezogen. Die ohne Zusatz eines Stimulators entstandene Menge "Gesamtprostaglandin" wurde f=1 gesetzt; die nach Stimulierung entstandene Menge wurde hierauf bezogen. f=2 oder f=3 bedeutet also doppelte oder dreifache Stimulierung unter den Bedingungen der Methode 2. Die Stabilität der Probe wurde mit Hilfe von Methode 1 untersucht (rechte Spalte). Ohne Zusätze (rechte Spalte: A) bleiben unter diesen Bedingungen in wässriger Lösung 25-28 % der Arachidonsäure erhalten. Werte größer als 25-28 % bedeuten also Stabilisierung, Werte kleiner als 25-28 % Destabilisierung. Beispiel Nr. 1 wurde ohne Stimulator durchgeführt. Die erfindungsgemäßen Effekte lassen sich mit den stabilisierten Zubereitungen der entsprechend bezeichneten Beispiele erhalten. Die hinsichtlich Stabilisierung geprüften Stoffe (Mengen siehe Methode 1) waren: A) wässrige Lösung ohne Stabilisatorzusatz, B) äthanolische Lösung ohne weiteren Zusatz, C) Pyridinlösung, D) mit Rutin, in Dimethylsulfoxid vorgelöst, dann Dimethylsulfoxid abgezogen, E) wie B) mit Lecithin, Äthanol abgezogen, F) wie A) mit Cytochrom c aus Pferdeherz (Fa. Boehringer, Mannheim, Deutschland), G) wie A) mit Methämoglobin (human) (Fa. Sigma, München, Deutschland), H) wie A) mit Serumalbumin vom Rind (Fa. Calbiochem, Frankfurt, Deutschland), J) wie A) oder B), jedoch Lösungsmittel abgezogen, K) wie J) mit Cytochrom c, L) wie J) mit Hämoglobin (human) (Fa. Behringwerke, Marburg, Deutschland), M) wie J) mit Humanserumalbumin (Fa. Behringwerke, Marburg, Deutschland).

| Beispiel Nr. | Stimulator | Arachidonsäure-Produkt (Methode 2): relatives Gesamtprostaglandin | PGE$_2$ | PGF$_{2alpha}$ | PGD$_2$ | Haltbarkeit (% verbleibende Arachidonsäure nach Methode 1) |
|---|---|---|---|---|---|---|
| | | f | % | % | % | |
| **Vergleichsuntersuchung** | | | | | | |
| 1) | ohne Stimulator | 1 | 83 | 2 | 15 | A 26<br>B 100<br>C 13<br>D 97<br>E 90<br>F 98<br>G 90<br>H 100<br>J 0<br>K 100<br>L 85<br>M 100 |
| **Beispiele nicht erfindungsgemäßer Substanzen** | | | | | | |
| 2) | m-Kresol | 0,61 | 98 | 0 | 2 | |
| 3) | Phenol | 0,95 | 77 | 5 | 18 | |
| 4) | L-Phenylalanin | 0,9 | 80 | 2 | 18 | |
| 5) | trans-Zimtsäure | 0,9 | | | | |
| **Beispiele erfindungsgemäßer Verbindungen der Formel I** | | | | | | |
| 6) | $R_1$ = $-CH_2-CH(CO_2H)-NH_2$<br>$R_2$ = $R_3$ = -H | 5,5 | 81 | 2 | 17 | A 91<br>B 100<br>C 67<br>J 98<br>K 100<br>M 100 |
| 7) | $R_1$ = $-CH_2-CH_2-NH_2$<br>$R_2$ = $R_3$ = -H | 4,0 | 80 | 3 | 17 | A 87 |
| 8) | $R_1$ = $-CH_2-CH_2-CO_2H$<br>$R_2$ = $R_3$ = -H | 4,5 | 83 | 1 | 16 | A 89 |

| | | | | | | | |
|---|---|---|---|---|---|---|---|
| 9) | $R_1 = -CH_2-\underset{CO_2H}{CH}-NH_2$<br>$R_2 = -OH$<br>$R_3 = -H$ | 9,4 | 70 | 14 | 16 | A 15<br>B 97<br>C 93 | F 97<br>G 96<br>M 100 |
| 10) | $R_1 = -CH_2-CH_2-NH_2$<br>$R_2 = -OH$<br>$R_3 = -H$ | 6,0 | 52 | 35 | 13 | A 17<br>B 95<br>C 9 | H 100<br>K 98<br>M 100 |
| 11) | $R_1 = -CH_2-\underset{CONH_2}{CH}-NH-\overset{O}{C}-CH_3$ | (Stimulierung: positiv) | | | | A (Stabilisierung: positiv) | |
| 12) | $R_1 = -CHOH-CH_2-NH_2$<br>$R_2 = -OH$<br>$R_3 = -H$ | 8,3 | 66 | 17 | 17 | A 11<br>B 93<br>D 93 | F 99<br>G 95<br>M 100 |
| 13) | $R_1 = -CHOH-CH_2-NHCH_3$<br>$R_2 = -OH$<br>$R_3 = -H$ | 13 | 70 | 12 | 18 | A 9<br>B 92<br>E 94 | H 97<br>J 0<br>M 100 |
| 14) | $R_1 = -OH$<br>$R_2 = R_3 = -H$ | 3,2 | 69 | 12 | 19 | A 7<br>D 97<br>F 98 | G 99<br>K 97<br>M 99 |
| 15) | $R_1 = -CO_2H$<br>$R_2 = -OCH_3$<br>$R_3 = -H$ | 5,1 | 71 | 12 | 17 | A 22<br>B 100<br>D 98 | F 98<br>G 96<br>J 17 |
| 16) | $R_1 = -CO_2H$<br>$R_2 = R_3 = -H$ | 4,5 | 69 | 13 | 18 | A 21<br>B 98<br>D 97 | F 98<br>G 96<br>H 99 |
| 17) | $R_1 = -CH=CHCO_2H$<br>$R_2 = -OH$<br>$R_3 = -H$ | 5,7 | 70 | 13 | 17 | A 19<br>D 99<br>G 95 | H 99<br>J 16<br>M 98 |
| Beispiele erfindungsgemäßer Verbindungen der Formel II | | | | | | | |
| 18) | $R_1 = -CO_2H$<br>$R_2 = -NH_2$<br>$R_3 = -H$ | 9,8 | 76 | 3 | 21 | A 18<br>D 99<br>G 99 | H 98<br>J 15<br>K 99[1)] |
| 19) | $R_1 = -CO_2H$<br>$R_2 = -NH_2$<br>$R_3 = -OH$ | 10,1 | 79 | 3 | 18 | A 17<br>B 95<br>D 96 | E 95<br>F 97<br>G 95 |
| 20) | $R_1 = -CO_2H$<br>$R_2 = R_3 = -F$ | 5,1 | 82 | 2 | 16 | A 20<br>B 100<br>E 96 | F 98<br>H 99<br>M 99 |

| | | | | | | | |
|---|---|---|---|---|---|---|---|
| 21) | $R_1$ = -$CO_2H$<br>$R_2$ = -H<br>$R_3$ = -OH | 5,7 | 80 | 3 | 17 | A 18<br>B 99<br>F 98 | G 96[2]<br>H 97<br>J 12 |
| 22) | $R_1$ = -H<br>$R_2$ = -$NH_2$<br>$R_3$ = -OH | 10,6 | 75 | 5 | 20 | A 16<br>B 99<br>E 96 | F 98<br>K 98<br>L 100 |
| 23) | $R_1$ = -H<br>$R_2$ = -$NH_2$<br>$R_3$ = -H | 6,1 | 81 | 3 | 16 | A 20<br>D 100<br>E 97[1] | F 98<br>G 97<br>H 100 |
| | **Beispiele erfindungsgemäßer Tyrosin-(Tyr-) bzw. Tryptophan-(Trp-) haltiger Peptide** | | | | | | |
| 24) | Trp-Trp | 11,7 | 75 | 2 | 23 | A 17<br>B 98<br>C 12 | D 97<br>F 97<br>G 96 |
| 25) | Gly-Trp-Gly | 13,0 | 79 | 1 | 20 | A 18<br>F 96<br>G 97 | H 99<br>J 7<br>K 99 |
| 26) | Trp-Ala | 12,6 | 77 | 1 | 22 | A 19<br>D 98 | E 92<br>F 96 |
| 27) | Leu-Trp | 17,0 | 77 | 1 | 22 | A 20<br>B 99 | F 98<br>M 100 |
| 28) | Trp-Met-Asp-Phe | 11,8 | 73 | 7 | 20 | A 17<br>D 97 | D 97[1]<br>F 96 |
| 29) | Leu-Trp-Met | 10,8 | 69 | 9 | 22 | A 18<br>B 99<br>D 97 | H 99<br>J 7<br>K 100 |
| 30) | Tyr-Tyr-Tyr-$OCH_3$ | 5,3 | 75 | 4 | 11 | A 94<br>B 98 | J 98<br>K 100 |
| 31) | Tyr-Trp | 6,3 | 73 | 3 | 14 | A 80<br>B 97 | D 98<br>M 100 |
| 32) | Cyclo-Trp-Tyr | (Stimulierung:positiv) | | | | (Stabilisierung:positiv) | |
| | **Beispiele erfindungsgemäßer schwefelhaltiger Verbindungen** | | | | | | |
| 33) | L-Cystein | 4,0 | 56 | 17 | 27 | A 9<br>B 95<br>D 94[3] | D 95<br>H 96<br>M 96 |
| 34) | D-Cystein | 3,5 | 63 | 14 | 23 | A 13<br>D 96<br>D 95 | H 97<br>J 5[2]<br>K 98 |

| | | | | | | | |
|---|---|---|---|---|---|---|---|
| 35) | Glutathion | 13,5 | 100 | 0 | 0 | A 0<br>B 80<br>C 0 | H 91<br>J 0<br>L 92 |
| 36) | Liponsäure | 5,5 | 43 | 49 | 8 | A 92<br>E 94<br>F 100 | G 100<br>J 90<br>L 100 |
| 37) | N-Acetylcystein | 4,0 | 61 | 14 | 25 | A 9<br>B 93<br>D 95 | E 93<br>F 98<br>G 98 |
| 38) | Tyr-Cys-Glu-His--Phe-Arg-Trp-Gly | (Stimulierung: positiv) | | | | M 100 | |
| Nicht erfindungsgemäße schwefelhaltige Verbindungen | | | | | | | |
| 39) | Dithiothreit | 0,8 | | | | | |
| 40) | Dithioerythrit | 0,85 | | | | | |
| 41) | Cystin | 1,0 | 85 | 0 | 15 | | |
| 42) | Dihydroliponsäure | 1,0 | | | | | |
| 43) | Cysteamin | 0,9 | | | | | |
| 44) | Mercaptoethanol | 0,95 | | | | | |
| 45) | Coenzym A | 1,0 | | | | | |
| 46) | Pantethein | 0,95 | | | | | |

1) - 3): statt Arachidonsäure wurde 1) 5,8,11,14,17-Eicosapentaensäure bzw. 2) 8,11,14-Eicosatriensäure bzw. 3) 9,12,15-Linolensäure eingesetzt.
4): Gamma-Globulin vom Hasen (Fa. Calbiochem, Frankfurt)

Tab.2

Untersuchungen der erfindungsgemäßen Eigenschaften von Vitaminen, Flavonoiden, Cyclodextrinen und (Zucker-)Lipid-Verbindungen. Die im Stabilisierungsexperiment eingesetzten Fettsäuren sind in Klammern angegeben. (A = Arachidonsäure, E = 5,8,11,14,17-Eicosapentaensäure)

| Beispiel Nr. | Stimulator | Arachidonsäure-Produkt (Methode 2) relatives Gesamt-prostaglandin | PGE2 | PGF2alpha | PGD2 | Stabilisierung (% verbleibende unges. Fettsäure nach Methode 1) |
|---|---|---|---|---|---|---|
| | | f | % | % | % | |
| Nicht erfindungsgemäße Vitamine | | | | | | |
| 47) | Vitamin Q10 | 0,8 | 8 | 78 | 14 | 8% (A) |
| 48) | Vitamin B12 | | | | | 6% (A) |
| 49) | Vitamin C | | | | | 0% (A) |
| Erfindungsgemäße Vitamine | | | | | | |
| 50) | DL-alpha-Tocopherol | | | | | 78% (A) |
| 51) | D-alpha-Tocopheryl-chinon | 0,5 | 80 | 5 | 15 | 98% (A) |
| Erfindungsgemäße zuckerhaltige Flavonoide | | | | | | |
| 52) | Rutin | 6 | 76 | 6 | 18 | 97% (A)<br>96% (E) |
| 53) | Phloridzin | 3 | 80 | 2 | 18 | 97% (E) |
| 54) | Naringin | 2 | 83 | 2 | 15 | 96% (A) |
| Erfindungsgemäße Flavonoide ohne Zuckeranteil | | | | | | |
| 55) | Naringenin | 1,5 | 80 | 0 | 20 | 96% (A) |
| 56) | Kämpferol | 0,5 | 76 | 11 | 13 | 98% (E) |

| | | | | | | |
|---|---|---|---|---|---|---|
| 57) | Quercetin | 0,3 | 72 | 18 | 10 | 92% (E) |
| 58) | Catechin | 0,2 | 70 | 18 | 12 | 90% (A) |
| 59) | Epicatechin | 0,15 | 71 | 17 | 12 | 89% (A) |
| 60) | Phloretin | 0,15 | 76 | 11 | 13 | 97% (A) |
| 61) | Morin | 0,08 | 54 | 27 | 19 | 100% (E) |

Alle untersuchten Flavonoide stabilisieren die mehrfach ungesättigten Fettsäuren. Erfindungsgemäß sind die zuckerhaltigen Flavonoide besonders geeignet, weil sie die Bildung der Prostaglandine stimulieren. Insbesondere für die erfindungsgemäße Anwendung in vitro sind die Flavonoide ohne Zuckeranteil weniger gut geeignet, weil sie die Prostaglandinsynthese hemmen.

Erfindungsgemäße Cyclodextrine

| | | |
|---|---|---|
| 62) | alpha-Dextrin (Cyclohexaamylose) | 60% (A) |
| 63) | beta-Dextrin (Cycloheptaamylose) | 90% (A)<br>88% (E) |
| 64) | gamma-Dextrin (Cyclooctaamylose) | 75% (A) |

Erfindungsgemäße Lipide (Fa.Serva, Heidelberg)

| | | |
|---|---|---|
| 65) | Sucrose palmitat-stearat 7 SPS-7 | 91% (A)<br>92% (E) |
| 66) | Sucrosepalmitat-stearat 15 SPS-15 | 92% (A) |
| 67) | Dioctylsulfosuccinat, Na-Salz | 94% (A) |

Beispiele stabiler erfindungsgemäßer Zubereitungen einschließlich pharmazeutischer Zubereitungen

Beispiele 68) bis 70)

Unter sterilen Bedingungen werden 50 mg Natriumarachidonat, 500 mg Serumalbumin vom Rind und 500 mg L-Tyrosin in 10 ml bidestilliertem Wasser gelöst, und, ebenfalls unter sterilen Bedingungen, in einer braunen Flasche aufbewahrt. 93,5% der ursprünglich vorhandenen Arachidonsäure werden nach 6 Wochen Aufbewahrung bei Zimmertemperatur wiedergefunden, während die gleiche Natriumarachidonatlösung ohne Zusätze nach dieser Zeit praktisch keine Arachidonsäure (Gehalt < 0,8%) mehr enthält. Die derart hergestellte stabilisierte Lösung wird

Beispiel 68) für medizinische Zwecke in eine Trinkampulle abgefüllt,

Beispiel 69) mit 15 mg Schafsamenblasenvesikeln versetzt, 60 min bei 37 °C inkubiert, und sodann die gebildeten Prostaglandine extrahiert und präparativ dargestellt wie in Methode 2 beschrieben,

oder

Beispiel 70) für die klinisch-biochemische Bestimmung der Thromboseneigung von Probanden (Patienten) verwendet, indem 0,01 ml dieser stabilisierten Natriumarachidonatlösung mit 1 ml lysiertem plättchenreichem Blutplasma dieser Probanden versetzt wird, und nach 30 min Inkubation bei 25 °C extrahiert wird. Das gebildete Prostaglandin $D_2$ (Methode 2) dient als Maß für die Thromboseneigung des Patienten.

Beispiel 71)

100 mg 5,8,11,14,17-Eicosapentaensäure und 500 mg Rutin werden zusammen in 15 ml Dimethylsulfoxid aufgenommen, das Dimethylsulfoxid restlos abgezogen und der Rückstand zusammen mit Acetylsalicylsäure-Kristallen (500 mg R 95 D, Röhm Pharma, Darmstadt, BR Deutschland) in Gelatinekapseln der Größe 0 (Fa. Kapsugel, Basel, Schweiz) überführt.

Beispiel 72)

In 10 ml bidestilliertes Wasser werden 1 g L-alpha-Lecithin, beta, gamma-dipalmitoyl (Fa. Calbiochem GmbH, Frankfurt/Main, Deutschland), 100 mg Arachidonsäure, 100 mg Liponsäure und 100 mg DL-alpha-Tocopherolacetat (Fa. Aldrich, Steinhelm, Deutschland) eingerührt und durch Ultrabeschallung homogenisiert.

Beispiel 73)

170 mg Weizenstärke, 450 mg Laktose, 20 mg Magnesiumstearat, 5 mg Natriumarachidonat, 10 mg Tryptophan und 20 mg Troxerutin (Fa. Aldrich, Steinheim, Deutschland) werden gemahlen, innig vermischt und zu einer Tablette gepreßt.

Beispiel 74)

150 mg Weizenstärke, 435 mg Laktose, 15 mg Magnesiumstearat, 5 mg Natriumarachidonat und 15 mg Liponsäure werden durch Mahlen innig vermischt und zu einer Tablette gepreßt.

Beispiel 75)

10 mg Natriumarachidonat werden mit 20 mg Serotonin und 100 mg Hämoglobin (reduziert, Fa. Calbiochem GmbH, Frankfurt) in 3,5 ml Wasser (bidestilliert) aufgenommen und lyophilisiert. Der Rückstand kann zu Dosiseinheiten in Kapseln (Gelatinekapseln Größe 0, Fa. Kapsugel, Basel, Schweiz) verarbeitet werden.

Beispiel 76)

15 mg 8,11,14-Eicosatriensäure werden mit 50 mg N-Acetylcystein und 100 mg Cytochrom c aus Pferdeherz (Fa. Aldrich, Steinheim, Deutschland) in 2 ml Wasser (bidestilliert) aufgenommen.

Beispiel 77)

40 mg Arachidonsäure werden unter sterilen Bedingungen mit 180 mg beta-Cyclodextrin und 60 mg 5-Hydroxytryptamin in 50 ml Wasser (bidestilliert) aufgenommen.

Beispiel 78 a-f)

Es werden 6 Ansätze a,b,c,d,e,f hergestellt, die in 1 ml Äthanol jeweils 0,7 mg Arachidonsäure und a) 1, b) 5, c) 10, d) 25, e) 50 bzw. f) 100 mg Liponsäure enthalten. Nach Abziehen des Äthanols können diese Ansätze mindestens 12 Tage bei 37 °C gehalten werden. Die Bestimmung entsprechend Methode 2 ergab in den Ansätzen nach 12 Tagen noch 99 ± 2% Arachidonsäure.

Beispiele 79) - 82)

0,125 mg Arachidonsäure werden in 0,25 ml Wasser, das 5 bzw. 25 bzw. 50 bzw. 75 Gewichtsprozent Dimethylsulfoxid enthält, aufgenommen und bei 37 °C inkubiert. Nach 24 Stunden erfolgt Extraktion und Analyse. Eine typische Analyse ergab

| | % Dimethylsulfoxid | % Arachidonsäure |
|---|---|---|
| Beispiel 79) | 5 | 54 |
| Beispiel 80) | 25 | 75 |
| Beispiel 81) | 50 | 83 |
| Beispiel 82) | 75 | 94 |

Beispiel 83)

In 5 ml Glycerintriacetat werden 2 g Arachidonsäure sowie 1 g Tyrosamin eingerührt und bei 30 °C durch Ultraschallbehandlung gleichmäßig verteilt.

Beispiel 84)

60 mg Arachidonsäure (Eicosapentansäure) werden mit der doppelten Gewichtsmenge eines der folgenden Stabilisatoren (I Betacyclodextrin, II Rinderserumalbumin oder III Liponsäure) in 2 ml Wasser, enthaltend 5 mmol/l Pluronic (Serva, Heidelberg, Deutschland) aufgenommen und eine Stunde bei 37 °C gehalten. Für Kontrollexperimente wurden entsprechende Lösungen ohne Stabilisator hergestellt und ebenfalls eine Stunde entweder bei 37 °C oder im Eisbad aufbewahrt. Diese Zubereitungen wurden mit Hilfe einer pharmakologischen Standardmethode (vgl. Gastroenterology 77, 1979, 433 f) auf cytoprotektive Wirkung an der Ratte untersucht. Durch Vorbehandlung der Ratten mit jeweils 0,1 ml/kg Körpergewicht der Zubereitung I oder II, wie auch der gekühlt gehaltenen Kontrollzubereitung, konnte die Geschwürbildung fast vollständig, und durch Zubereitung III vollständig verhindert werden, während die eine Stunde bei 37 °C gehaltene Zubereitung keine geschwürhemmenden Effekte zeigte.

**Patentansprüche**

1. Zubereitungen für die Synthese von Prostaglandinen und Hydroxyfettsäuren in biologischen Systemen, dadurch gekennzeichnet, daß sie enthalten:
   A) eine oder mehrere ungesättigte Fettsäure(n), gekennzeichnet durch drei bis fünf isoliert angeordnete Doppelbindungen und 18 bis 22 geradkettig angeordnete Kohlenstoffatome, die an einem oder an zwei C-Atomen der Position 2, 3, 4, 16, 17, 18, 19 oder 20, methyliert oder äthyliert sein können, als freie Carbonsäure mit endständigem $-CO_2H$ oder als Carbonsäureamid oder $-CO_2X$, wobei X eine unter sauren Bedingungen abspaltbare Schutzgruppe oder ein 1- oder 2-Lysophospholipid, oder ein Metall- oder Amin-Kation oder die kationische Form eines Ionenaustauschers darstellt, und
   B) als Stimulatoren mit zum Teil gleichzeitig stabilisierender Wirkung eine oder mehrere Verbindungen der folgenden Gruppen:
   $B_1$) eine phenolische Verbindung der Formel I

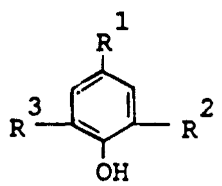

I

in der die Reste $R^2$ und $R^3$ Hydroxylgruppen oder Wasserstoff, und $R^1$ den Rest -OH, $-CO_2H$,-$CH_2$-COOH, $-CH=CH-CO_2H$, $-CH_2-CHR^4R^5$, $-CHOH-CH_2-NH-R^6$ mit $R^4$ = -H oder $-CO_2H$ und $R^5$ = -H oder $-NH_2$ und $R^6$ = -H, $-CH_3$ oder $-C_2H_5$ bedeuten,
$B_2$) ein Indolderivat der Formel II

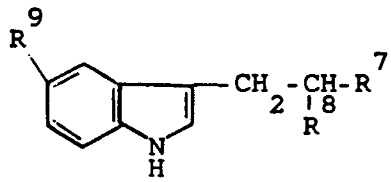

II

in der $R^7$ Wasserstoff oder -COOH, $R^8$ Wasserstoff oder $-NH_2$ und $R^9$ Wasserstoff oder -OH bedeuten,

$B_3$) Cystein Homocystein oder Liponsäure, deren acyclischer Alkylrest um bis zu vier Methylengruppen verkürzt sein kann,

$B_4$) ein Peptid, bestehend aus maximal zehn Aminosäuren, bei dem eine oder mehrere der Aminosäuren durch jeweils eine der Verbindungen gemäß $B_1$) bis $B_3$) ersetzt ist,

$B_5$) eine Aminoverbindung gemäß $B_1$) bis $B_4$), worin ein N-Atom substituiert sein kann mit einer $C_1$-$C_4$-Alkanoylgruppe,

$B_6$) einen Flavonabkömmling, der mit wenigstens einer Hydroxylgruppe, die einen Zuckerrest trägt , substituiert ist,

$B_7$) ein Salz der ionischen Formen der Verbindungen gemäß $B_1$)-$B_6$), und gegebenenfalls

$B_8$) eine Carbonsäureverbindung gemäß $B_1$) bis $B_7$), die mit einem Alkoxy-Rest verestert sein kann oder als Carbonsäureamid vorliegt, das auch mono- oder dialkyliert sein kann; und gegebenenfalls

C) als Stabilisatoren eine oder mehrere Verbindungen der folgenden Gruppen:

$C_1$) Dimethylsulfoxid, Äthanol, Polyole oder Polyolester,

$C_2$) Phospholipide, Zuckerlipide, Cyclodextrine, Proteine, Cytochrome der C-Reihe, Vitamine der E-Reihe oder Flavonabkömmlingen ohne Zuckerrest, in fester oder flüssiger Form.

**2.** Zubereitungen gemäß Anspruch 1, dadurch gekennzeichnet, daß die als Stabilisatoren der Gruppe $C_1$) zu verwendenden Polyole bzw. Polyolester Glycerin, Äthylenglykol, Polyäthylenglykol bzw. Glycerintriacetat sind.

**3.** Zubereitungen gemäß Anspruch 1, dadurch gekennzeichnet, daß sie als Fettsäuren der Gruppe A) 18:2ω-6, 20:4ω-6, 22:5ω-6, 18:3ω-3, 20:5ω-3, 22:6ω-3, 18:3ω-6, 20:3ω-6, 22:4ω-6 oder 22:4ω-3-Fettsäuren, als Stimulatoren der Gruppe B) Verbindungen der Formel I, worin $R^2$ und $R^3$ = Wasserstoff und $R^1$ den Rest $-CH_2-CH(NH_2)COOH$ bedeuten oder Peptide, die solche Verbindungen enthalten, oder Liponsäure, deren Seitenkette um bis zu 4 $CH_2$-Gruppen verkürzt sein kann, oder einen Flavonabkömmling enthalten und ggfs. kein Zusatz eines Stabilisators der Gruppe C) erforderlich ist.

**4.** Zubereitung gemäß Anspruch 1, dadurch gekennzeichnet, daß die Stabilisatoren der Gruppe C) Betadextrin, Lezithin, aus Human- oder Säugetierblut herstellbare Proteine oder Cytochrome der C-Reihe sind.

**5.** Zubereitungen gemäß Anspruch 1 und 3, dadurch gekennzeichnet, daß als zuckerhaltige Flavonabkömmlinge Rutin oder Troxerutin verwendet werden.

**6.** Zubereitungen gemäß Anspruch 4, dadurch gekennzeichnet, daß sie als Stabilisatoren der Gruppe C) Hämoglobin- oder Methämoglobin, Globulin oder Serumalbumin enthalten.

**7.** Zubereitungen gemäß Anspruch 1-6, dadurch gekennzeichnet, daß sie als Fettsäuren der Gruppe A) Arachidonsäure, 5,8,11,14,17-Eicosapentaensäure, 8,11,14-Eicosatriensäure oder 9,12,15-Linolensäure enthalten.

**8.** Zubereitungen gemäß Anspruch 1-7, dadurch gekennzeichnet, daß das Gewichtsverhältnis zwischen ungesättigter Fettsäure der Gruppe A) und Stimulator der Gruppe B) zwischen 50 und 0,1 liegt.

**9.** Zubereitungen gemäß Anspruch 8, dadurch gekennzeichnet, daß das Gewichtsverhältnis zwischen 20 und 0,5 liegt.

**10.** Zubereitungen gemäß Anspruch 1-7, dadurch gekennzeichnet, daß das Gewichtsverhältnis zwischen

ungesättigter Fettsäure der Gruppe A) und Stabilisator der Gruppe C) zwischen 100 und 0,5 liegt.

**11.** Zubereitungen gemäß Anspruch 10, dadurch gekennzeichnet, daß das Gewichtsverhältnis zwischen 15 und 1 liegt.

**12.** Zubereitungen gemäß Anspruch 1-11, dadurch gekennzeichnet, daß sie in einer dermal, oral, peroral oder als Suppositorien anzuwendenden Form vorliegen.

**13.** Zubereitungen gemäß Anspruch 12, dadurch gekennzeichnet, daß sie pro Dosierungseinheit zwischen 0,5 und 2000 mg, bevorzugt zwischen 5 und 250 mg an ungesättigter Fettsäure enthalten.

**14.** Zubereitungen gemäß Anspruch 1-13, dadurch gekennzeichnet, daß die ungesättigten Fettsäuren wenigstens teilweise in retardierter Form vorliegen.

**15.** Zubereitungen gemäß Anspruch 1, dadurch gekennzeichnet, daß sie eine oder mehrere Verbindungen aus der Gruppe A), eine oder mehrere Verbindungen aus der Gruppe B), soweit sie stabilisierende Wirkung besitzen, und gegebenenfalls eine oder mehrere Verbindungen aus der Gruppe C) enthalten.

**16.** Verwendung einer Zubereitung gemäß Anspruch 1-5 zur Herstellung eines Arzneimittels zur Behebung von Prostaglandinmangelzuständen bei Menschen oder Tieren.

**17.** Verwendung einer Zubereitung gemäß Anspruch 16, dadurch gekennzeichnet, daß das Arzneimittel zur Heilung oder Verhinderung von Erkrankungen des Gastrointestinaltrakts verwendet wird.

**18.** Arzneimittelpackung, mit der die Substanzen nach Anspruch 1 bis 15 getrennt, aber mindestens eine Substanz getrennt und andere in Verbindung miteinander, aber zu gemeinsamer Verabreichung, dargeboten werden.

## Claims

**1.** A formulation for the synthesis of prostaglandins and hydroxy-fatty acids in biological systems, which comprises:

A) one or more unsaturated fatty acid(s), defined by three to five isolated double bonds and 18 to 22 carbon atoms which are arranged in a straight chain and can be methylated or ethylated at one or two carbon atoms in positions 2, 3, 4, 16, 17, 18, 19 or 20, as the free carboxylic acid with terminal $-CO_2H$, or as the carboxamide or as $-CO_2X$, X being a protective group which can be eliminated under acidic conditions or a 1- or 2-lysophospholipid or a metal or amine cation or the cationic form of an ion exchanger, and

B) as stimulators with, in some cases, a simultaneous stabilizing action, one or more compounds from the following groups:

$B_1$) a phenolic compound of the formula I

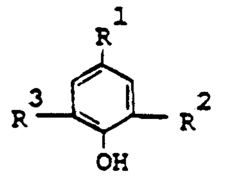

I

in which the radicals $R^2$ and $R^3$ are hydroxyl groups or hydrogen and $R^1$ is a radical $-OH$, $-CO_2H$, $-CH_2-COOH$, $-CH=CH-CO_2H$, $-CH_2-CHR^4R^5$ or $-CHOH-CH_2-NH-R^6$ with $R^4$ = $-H$ or $-CO_2H$ and $R^5$ = $-H$ or $-NH_2$ and $R^6$ = $-H$, $-CH_3$ or $-C_2H_5$,

$B_2$) an indole derivative of the formula II

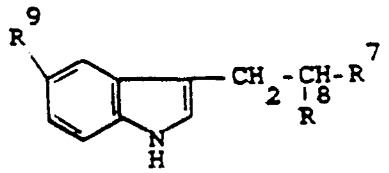

II

in which $R^7$ is hydrogen or -COOH, $R^8$ is hydrogen or $-NH_2$ and $R^9$ is hydrogen or -OH,

$B_3$) cysteine, homocysteine or lipoic acid, the acyclic alkyl radical of which can be shortened by up to four methylene groups,

$B_4$) a peptide comprising a maximum of ten amino acids, wherein one or more of the amino acids are replaced by one of the compounds according to $B_1$) to $B_3$) in each case,

$B_5$) an amino compound according to $B_1$) to $B_4$), wherein one N atom can be substituted by a $C_1$-$C_4$-alkanoyl group,

$B_6$) a flavone derivative which is substituted by at least one hydroxyl group which carries a sugar radical,

$B_7$) a salt of the ionic forms of the compounds according to $B_1$) - $B_6$) and, if appropriate,

$B_8$) a carboxylic acid compound according to $B_1$) to $B_7$) which can be esterified with an alkoxy radical or be in the form of the carboxamide which can also be mono- or di-alkylated, and, where appropriate,

C) as stabilizers, one or more compounds from the following groups:

$C_1$) dimethyl sulfoxide, ethanol, polyols or polyol esters and

$C_2$) phospholipids, sugar lipids, cyclodextrins, proteins, cytochromes of the C series, vitamins of the E series or flavone derivatives without a sugar radical, in the solid or liquid form.

**2.** A formulation as claimed in claim 1, wherein the polyols or polyol esters to be used as stabilizers of group $C_1$) are glycerol, ethylene glycol, polyethylene glycol or glycerol triacetate respectively.

**3.** A formulation as claimed in claim 1, which comprises, as the fatty acids of group A), 18:2 $\omega$-6, 20:4 $\omega$-6, 22:5 $\omega$-6, 18:3 $\omega$-3, 20:5 $\omega$-3, 22:6 $\omega$-3, 18:3 $\omega$-6, 20:3 $\omega$-6, 22:4 $\omega$-6 or 22:4 $\omega$-3 fatty acids and, as the stimulators of group B), compounds of the formula I, wherein $R^2$ and $R^3$ are hydrogen and $R^1$ is a radical $-CH_2-CH(NH_2)COOH$ or peptides which contain such compounds, or lipoic acid, the side chain of which can be shortened by up to 4 $CH_2$ groups, or a flavone derivative, no addition of a stabilizer from group C) being necessary in some cases.

**4.** A formulation as claimed in claim 1, wherein the stabilizers of group C) are betadextrin, lecithin, proteins which can be prepared from human or mammal blood, or cytochromes of the C series.

**5.** A formulation as claimed in claim 1 or 3, wherein rutin or troxerutin are used as the sugar-containing flavone derivatives.

**6.** A formulation as claimed in claim 4, which comprises, as the stabilizers of the group C), hemoglobin or methemoglobin, globulin or serum albumin.

**7.** A formulation as claimed in any of claims 1 - 6, which comprises, as the fatty acids of group A), arachidonic acid, 5,8,11,14,17-eicosapentaenoic acid, 8,11,14-eicosatrienoic acid or 9,12,15-linolenic acid.

**8.** A formulation as claimed in any of claims 1 - 7, wherein the weight ratio of unsaturated fatty acid from group A) and stimulator from group B) is between 50 and 0.1.

**9.** A formulation as claimed in claim 8, wherein the weight ratio is between 20 and 0.5.

**10.** A formulation as claimed in any of claims 1 - 7, wherein the weight ratio of unsaturated fatty acid from group A) and stabilizer from group C) is between 100 and 0.5.

**11.** A formulation as claimed in claim 10, wherein the weight ratio is between 15 and 1.

**12.** A formulation as claimed in any of claims 1 - 11, which is in a form to be administered dermally, orally, perorally or as suppositories.

**13.** A formulation as claimed in claim 12, which contains, per dosage unit, between 0.5 and 2,000 mg, preferably between 5 and 250 mg, of unsaturated fatty acid.

**14.** A formulation as claimed in any of claims 1 - 13, wherein the unsaturated fatty acids are at least partially in a retarded form.

**15.** A formulation as claimed in claim 1, which comprises one or more compounds from group A), one or more compounds from group B) if they have a stabilizing action, and, where appropriate, one or more compounds from group C).

**16.** The use of a formulation as claimed in any of claims 1 - 15 for the preparation of a medicament for curing prostaglandin deficiencies in humans or animals.

**17.** The use of a formulation, as claimed in claim 16, wherein the medicament is used for curing or preventing diseases of the gastro-intestinal tract.

**18.** A medicament package, wherein the substances as claimed in any of claims 1 to 15 are provided separately, at least one substance being separate and others being combined, but to be administered together.

**Revendications**

**1.** Compositions pour la synthèse de prostaglandines et d'acides gras hydroxylés dans des systèmes biologiques, caractérisées en ce qu'elles contiennent

A) un ou plusieurs acide(s) gras insaturé(s), caractérisé(s) par trois à cinq doubles liaisons isolées et 18 à 22 atomes de carbone en chaîne droite, qui peut(peuvent) être méthylé(s) ou éthylé(s) sur un ou sur deux atomes de carbone en position 2, 3, 4, 16, 17, 18, 19 ou 20, sous forme d'acide carboxylique libre à groupe $-CO_2H$ terminal ou sous forme d'amide ou de groupe $-CO_2X$, X représentant un groupe protecteur séparable dans des conditions acides ou un 1- ou 2-lysophospholipide ou un cation métallique ou d'amine, ou la forme cationique d'un échangeur d'ions, et

B) en tant que stimulants à action parfois simultanément stabilisante, un ou plusieurs composés appartenant aux groupes suivants:

$B_1$) un composé phénolique de formule I

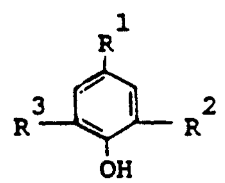

I

dans laquelle les radicaux $R^2$ et $R^3$ représentent des groupes hydroxy ou un atome d'hydrogène, et $R^1$ représente le radical -OH, $-CO_2H$, $-CH_2-COOH$, $-CH=CH-CO_2H$, $-CH_2-CHR^4R^5$, $-CHOH-CH_2-NH-R^6$, avec $R^4$ = -H ou $-CO_2H$ et $R^5$ = -H ou $-NH_2$ et $R^6$ = -H, $-CH_3$ ou $-C_2H_5$,

$B_2$) un dérivé d'indole de formule II

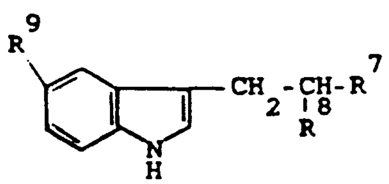

II

dans laquelle $R^7$ représente un atome d'hydrogène ou -COOH, $R^8$ représente un atome d'hydrogène ou $-NH_2$ et $R^9$ représente un atome d'hydrogène ou -OH,

$B_3$) la cystéine, l'homocystéine ou l'acide lipoïque, dont le radical alkyle acyclique peut être raccourci de jusqu'à 4 groupes méthylène,

$B_4$) un peptide constitué d'au maximum 10 aminoacides, dans lequel un ou plusieurs des aminoacides est(sont) remplacé(s) dans chaque cas par l'un des composés selon $B_1$) à $B_3$),

$B_5$) un composé aminé selon $B_1$) à $B_4$), dans lequel un atome d'azote peut être substitué par un groupe alcanoyle en $C_1$-$C_4$,

$B_6$) un dérivé de flavone qui est substitué par au moins un groupe hydroxy qui porte un reste glucidique,

$B_7$) un sel des formes ioniques des composés selon $B_1$)-$B_6$), et éventuellement

$B_8$) un composé de type acide carboxylique selon $B_1$) à $B_7$), qui peut être estérifié par un radical alcoxy ou se trouve sous forme d'amide qui peut également être mono- ou dialkylé; et éventuellement

C) en tant que stabilisants, un ou plusieurs composés appartenant aux groupes suivants:

$C_1$) le diméthylsulfoxyde, l'éthanol, des polyols ou des polyol-esters,

$C_2$) des phospholipides, glycolipides, cyclodextrines, protéines, cytochromes de la série c, vitamines de la série E ou des dérivés de flavone sans reste glucidique, sous forme solide ou liquide.

**2.** Compositions selon la revendication 1, caractérisées en ce que les polyols ou polyol-esters à utiliser en tant que stabilisants du groupe $C_1$) sont le glycérol, l'éthylèneglycol, le polyéthylèneglycol ou le triacétate de glycérol.

**3.** Compositions selon la revendication 1, caractérisées en ce qu'elles contiennent, en tant qu'acides gras du groupe A), des acides gras 18:2ω-6, 20:4ω-6, 22:5ω-6, 18:3ω-3, 20:5ω-3, 22:6ω-3, 18:3ω-6, 20:3ω-6, 22:4ω-6 ou 22:4ω-3, en tant que stimulants du groupe B), des composés de formule I dans lesquels $R^2$ et $R^3$ = H et $R^1$ représente le radical $-CH_2-CH(NH_2)COOH$ ou des peptides qui contiennent de tels composés, ou l'acide lipoïque, dont la chaîne latérale peut être raccourcie de jusqu'à 4 groupes $CH_2$, ou un dérivé de flavone, et éventuellement aucun additif d'un stabilisant du groupe C) n'est nécessaire.

**4.** Composition selon la revendication 1, caractérisée en ce que les stabilisants du groupe C) sont la $\beta$-dextrine, la lécithine, des protéines pouvant être obtenues à partir de sang humain ou d'autres mammifères, ou des cytochromes de la série c.

**5.** Compositions selon les revendications 1 et 3, caractérisées en ce que, en tant que dérivés glucidiques de flavone, on utilise la rutine ou la troxérutine.

**6.** Compositions selon la revendication 4, caractérisées en ce qu'elles contiennent, en tant que stabilisants du groupe C), de l'hémoglobine ou de la méthémoglobine, de la globuline ou de l'albumine de sérum.

**7.** Compositions selon les revendications 1-6, caractérisées en ce qu'elles contiennent, en tant qu'acides gras du groupe A), de l'acide arachidonique, de l'acide 5,8,11,14,17-eicosapentaénoïque, de l'acide 8,11,14-eicosatriénoïque ou de l'acide 9,12,15-linolénique.

**8.** Compositions selon les revendications 1-7, caractérisées en ce que le rapport pondéral entre acide gras insaturé du groupe A) et stimulant du groupe B) est compris entre 50 et 0,1.

**9.** Compositions selon la revendication 8, caractérisées en ce que le rapport pondéral est compris entre 20 et 0,5.

**10.** Compositions selon les revendications 1-7, caractérisées en ce que le rapport pondéral entre acide gras insaturé du groupe A) et stabilisant du groupe C) est compris entre 100 et 0,5.

**11.** Compositions selon la revendication 10, caractérisées en ce que le rapport pondéral est compris entre 15 et 1.

**12.** Compositions selon les revendications 1-11, caractérisées en ce qu'elles se trouvent sous une forme utilisable par voie dermique, orale, perorale ou sous forme de suppositoires.

**13.** Compositions selon la revendication 12, caractérisées en ce qu'elles contiennent, par dose unitaire, entre 0,5 et 2 000 mg, de préférence entre 5 et 250 mg, d'acide gras insaturé.

**14.** Compositions selon les revendications 1-13, caractérisées en ce que les acides gras insaturés se trouvent au moins en partie sous forme retard.

**15.** Compositions selon la revendication 1, caractérisées en ce qu'elles contiennent un ou plusieurs composés du groupe A) un ou plusieurs composés du groupe B), dans la mesure où ils ont une activité stabilisante, et éventuellement un ou plusieurs composés du groupe C).

**16.** Utilisation d'une composition selon les revendications 1-5, pour la fabrication d'un médicament pour le traitement d'états déficients en prostaglandines chez l'homme ou l'animal.

**17.** Utilisation d'une composition selon la revendication 16, caractérisée en ce que l'on utilise le médicament pour remédier ou empêcher des maladies du tractus gastrointestinal.

**18.** Conditionnement de médicament, avec lequel les substances selon les revendications 1 à 15 sont présentées séparément, ou au moins une substance est présentée séparément et les autres le sont en association entre elles, mais pour administration simultanée.